# NEUE EUROPÄISCHE PATENTSCHRIFT

(11) **EP 0 412 381 B2**
(45) Veröffentlichungstag und Bekanntmachung des Hinweises auf die Entscheidung über den Einspruch: **25.02.2004**
(45) Hinweis auf die Patenterteilung: 03.05.2000
(21) Anmeldenummer: 90114532.6
(22) Anmeldetag: 28.07.1990
(51) Int. Cl.: C12N 15/82, C12N 15/56, A01N 63/00

(54) **Verwendung von Lysozym gen-Konstruktionen in Pflanzen zur Resistenzerhöhung**
Use of lysozyme gene constructions in plants for enhanced resistance
Utilisation dans des plantes de constructions du gène de lysozyme pour obtenir une résistance élevée

(30) Priorität: 10.08.1989 DE 3926390
(43) Veröffentlichungstag der Anmeldung: 13.02.1991
(73) Patentinhaber: BAYER AG, 51368 Leverkusen (DE)
(72) Erfinder: Hain, Rüdiger, Dr., D-4018 Langenfeld (DE); Stenzel, Klaus, Dr., D-4000 Düsseldorf 13 (DE)

(56) Entgegenhaltungen:
- EP-A- 0 184 288
- WO-A-89/04371
- BIOL. CHEM. HOPPE SEYLER, Band 370, 1989, Seite 888, Herbsttagung der Gesellschaft für Biologische Chemie, Osnabrick, DE; K. DÜRING et al.: "Synthesis, assembly and targetting of foreign chimeric proteins in transgenic Nicotiana tabacum cells"
- EUROPEAN JOURNAL OF CELL BIOLOGY, Band 50, 1989, Seiten 230-234, Wissenschaftliche Verlagsgesellschaft mbH, Stuttgart, DE; S. HIPPE et al.: "In situ localization of a foreign protein in transgenic plants by immunoelectron microscopy following high pressure freezing. Freeze substitution and low temperature embedding"
- UCLA SYMP. MOL. CELL. BIOL., NEW SER., Band 22, 1985, CELLULAR AND MOLECULAR BIOLOGY OF PLANT STRESS, 1985, pages 247-262, Alan R. Liss, Inc.; T. BOLLER: "Induction of hydrolases as a defense reaction against pathogens"

## Beschreibung

Die vorliegende Erfindung betrifft die Verwendung von Lysozym Gen-Konstruktionen in Pflanzen zur Erhöhung der Resistenz von Pflanzen gegenüber Pilzen und tierischen Schädlingen.

Ein großer Teil der Welternte von Kulturpflanzen wird ständig durch Schädlinge vernichtet (1967 betrug der Verlust an potentieller Ernte 35 %; vgl. Chemistry of Pesticides, herausgegeben von K.H. Büchel, John Wiley & Sons, New York, 1983, Seite 6). Es besteht somit ein dringendes Bedürfnis alle Möglichkeiten zu erforschen und nutzbar zu machen, welche geeignet sind, den Schädlingsbefall bei Kulturpflanzen zu vermindern oder zu verhindern.

In der Patentanmeldung WO 89/04371 wird die Transformation von Pflanzen mit bestimmten Lysozym Genen zur Erhöhung der Resistenz gegen bestimmte Bakterien beschrieben.

Es wurde nun gefunden, daß man eine erhöhte Resistenz von Pflanzen gegen Pilze und tierische Schädlinge erreichen kann, wenn man in die Erbmasse der Pflanzen (das Genom) eine oder mehrere (vorzugsweise eine) Lysozym Gen-Konstruktionen einbaut, die Lysozyme exprimieren und welche dadurch gekennzeichnet sind, daß sie aus chimären Genfusionen aus dem TR-Promotor, der Signalpeptid Sequenz von alpha- Amylase aus Gerste und einem oder mehreren (vorzugsweise einem) Lysozym Genen bestehen oder diese chimären Genfusionen enthalten.

Eine solche chimäre Genfusion und ihre Verwendung zur Transformation von Tabak (Nicotiana tabacum) ist bereits bekannt aus K. Düring, Dissertation, Universität Köln, 1988, "Wundinduzierbare Expression und Sekretion von T4 Lysozym und monoklonalen Antikörpern in Nicotiana Tabacum". In dieser Dissertation wurde darauf hingewiesen, daß das T4 Lysozym als antibakterielles Resistenzprotein in Pflanzen untersucht werden soll.

Es war bei Kenntnis des Standes der Technik überraschend und nicht vorhersehbar, daß mit Hilfe der erfindungsgemäß verwendbaren Gen-Konstruktionen eine besonders ausgeprägte Schaderregerresistenz von Pflanzen gegen Pilze und tierische Schädlinge erzielt werden kann.

Der Begriff Lysozyme beschreibt eine Gruppe von Enzymen EC 3.2.1.17, welche in der Natur vorkommen. Als Beispiele seien Hühnereiweiß-Lysozym und T4 Phagen-Lysozym genannt.

Unter Lysozym-Genen soll jede Nukleinsäure (DNA) verstanden werden, die nach ihrer Transkription in RNA und Translation in Protein (in einer geeigneten Umgebung) die Bildung eines Enzyms bewirkt, welches die bekannten Eigenschaften von Lysozymen besitzt.

Die Lysozym Gen-Konstruktionen oder ihre Komponenten können z. B. an ihrem Anfang und/oder Ende, noch DNA-Sequenzen enthalten, die ihre Funktion nicht oder nicht wesentlich behindern.

Die Lysozym Gene sowie die übrigen Komponenten der Lysozym Gen-Konstruktionen können in der Form vorliegen, wie sie im Genom ihrer Herkunftsorganismen enthalten sind ("genomische" Form, einschließlich nicht Lysozyme kodierender und/oder nicht regulatorisch wirkender Sequenzen (wie Introns) oder in einer Form, welche der cDNA ("copy" DNA) entspricht, die über mRNA mit Hilfe von Reverse-Transkriptase/Polymerase erhältlich ist (und keine Introns mehr enthält).

In den erfindungsgemäß verwendbaren Lysozym Gen-Konstruktionen können DNA-Abschnitte durch im wesentlichen gleichwirkende andere DNA-Abschnitte ersetzt sein.

Auch können sie an den Enden solche DNA-Sequenzen tragen, welche für die Handhabung der Gene jeweils angepaßt sind (z.B. "Linker").

Bevorzugte erfindungsgemäß verwendbare Lysozym Gen-Konstruktionen bestehen aus bzw. enthalten chimäre Genfusionen, die außer dem oder den Lysozym Genen, den TR-Promotor und die Signalpeptid Sequenz der alpha-Amylase aus Gerste enthalten, wie sie im Plasmid pSR 2-4 vorliegen.

Erfindungsgemäß bevorzugte Lysozym Gen-Konstruktionen enthalten das Hühnereiweiß-Lysozym-Gen und/oder das T4 Phagen-Lysozym-Gen. Besonders bevorzugt wird das T4 Phagen-Lysozym Gen. Ganz besonders bevorzugt wird das T4 Phagen-Lysozym-Gen, wie es im Plasmid pSR 2-4 vorliegt, sowie die im wesentlichen gleichwirkenden DNA-Sequenzen, erfindungsgemäß verwendet.

Besonders hervorgehoben sei die Verwendung der chimären Genfusion aus dem TR-Promotor, der Signalpeptid Sequenz von alpha-Amylase aus Gerste und der proteincodierten Region des T4 Phagen Lysozym Gens, wie sie im Plasmid pSR 2-4 enthalten ist.

Der Abschnitt mit der Genfusion, welche aus dem T4 Phagen-Lysozym Gen, dem TR-Promotor und der Signalpeptid Sequenz von alpha-Amylose aus Gerste besteht bzw. diese Genfusion enthält, kann im Bedarfsfall nach den üblichen Methoden mit Hilfe von Restriktionsenzymen aus dem Plasmid pSR 2-4 gewonnen werden.

Die Lysozym Gene können in den Gen-Konstruktionen vollständig, also mit ihren natürlichen regulatorisch wirkenden Teilen (insbesondere Promotoren) oder nur in Form der Strukturgene, welche das Protein Lysozym kodieren, vorliegen.

Der Escherichia coli Stamm TBl pSR 2-4, der das Plasmid pSR 2-4 enthält, wurde bei der Deutschen Sammlung von Mikroorganismen (DSM), Mascheroder Weg 1 b, D-3300 Braunschweig, Bundesrepublik Deutschland in Übereinstimmung mit den Bestimmungen des Budapester Vertrages über die internationale Anerkennung der Hinterlegung von Mikroorganismen für die Zwecke von Patentverfahren hinterlegt und hat die Hinterlegungsnummer DSM 5455 (Hinterlegungsdatum: 25. Juli 1989).

Erfindungsgemäß können Resistenzen, bzw. erhöhte Resistenzen erzielt werden gegen Schädlinge, insbesondere gegen Pilze und tierische Schädlinge, insbesondere Arthropoden wie Insekten und Milben sowie Nematoden. Besonders hervorgehoben werden hierbei die phytopathogenen Pilze.

Zu den schädlichen Insekten gehören insbesondere Insekten der Ordnungen:
Orthoptera, Dermaptera, Isoptera, Thysanoptera, Heteroptera, Homoptera, Lepidoptera, Coleoptera, Hymenoptera und Diptera.

Zu den schädlichen Milben gehören insbesondere:
Tarsonemus spp., Panonychus spp. und Tetranychus spp.

Zu den schädlichen Nematoden gehören insbesondere:
Pratylenchus spp., Heterodera spp. und Meloidogyne spp.

Zu den phytopathogenen Pilzen gehören insbesondere:
Plasmodiophoromycetes, Oomycetes, Chytridiomycetes, Zygomycetes, Ascomycetes, Basidiomycetes, Deuteromycetes.

Beispielhaft aber nicht begrenzend seien einige spezielle Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola:
Peronospora-Arten, wie beispielsweise Peronospora pisi oder P. brassicae;
Erysiphe Arten, wie beispielsweise Erysiphe graminis; Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria longipes;
Pseudocercosporella-Arten, wie beispielweise Pseudocercosporella herpotrichoides.
Weiterhin sei Helminthosporium carbonum aufgeführt.

Erfindungsgemäß kann praktisch bei allen Pflanzen eine Resistenz bzw. eine erhöhte Resistenz gegenüber den obigen Schädlingen verliehen werden. Ein besonderes Bedürfnis zur Resistenzerzeugung besteht naturgemäß bei den Kulturpflanzen, wie Forstpflanzen, z.B. Fichten, Tannen, Douglasien, Kiefern, Lärchen, Buchen und Eichen sowie Nahrungsmittel und Rohstoffe liefernde Pflanzen, z.B. Getreide (insbesondere Weizen, Roggen, Gerste, Hafer, Hirse, Reis und Mais), Kartoffel, Hülsenfrüchte, Sojabohnen, Erdnuß, Gemüse (insbesondere Kohlarten und Tomaten), Obst (insbesondere Äpfel, Birnen, Kirschen, Weintrauben, Citrus, Ananas und Bananen), Ölpalmen, Tee-, Kakao- und Kaffeesträucher, Tabak, Sisal und Baumwolle sowie bei Heilpflanzen, wie Rauwolfia und Digitalis.

Wie bereits angedeutet, werden erfindungsgemäß die Lysozym Gen-Konstruktionen ein- oder mehrfach (an gleichen oder verschiedenen Stellen des Genoms) in das natürliche pflanzliche Genom eingebaut. Bei Pflanzen, welche bereits über die Fähigkeit der Lysozymsynthese verfügen, kann der Einbau eines oder mehrerer zusätzlicher, gegebenenfalls "fremder" Lysozym Gene zu einem erheblich verbesserten Resistenzverhalten führen.

Wie bereits erwähnt wurde, ist die erhöhte Resistenz der gemäß der vorliegenden Erfindung transformierten Pflanzenzellen und Pflanzen von Bedeutung für Landwirtschaft und Forsten, für den Zierpflanzenanbau, den Heilpflanzenanbau und die Pflanzenzucht. Auch bei der Kultivierung von Pflanzenzellen, z.B. zur Gewinnung von pharmazeutisch brauchbaren Stoffen, ist es von Vorteil, Pflanzenzellen verfügbar zu haben, welche insbesondere gegen Pilze erhöhte Resistenzen aufweisen.

Die vorliegende Erfindung betrifft somit auch ein Verfahren zur Erzeugung transformierter Pflanzenzellen (einschließlich Protoplasten), ausgenommen Pflanzenzellen von Tabak (Nicotiana tabacum), und Pflanzen (einschließlich Pflanzenteile und Samen), ausgenommen Tabakpflanzen (Nicotiana tabacum), mit einer erhöhten Resistenz gegen Pilze und tierische Schädlinge, welches dadurch gekennzeichnet ist, daß man
(a) ein oder mehrere Lysozym Gen-Konstruktionen die aus chimären Genfusionen aus dem TR-Promotor, der Signalpeptid Sequenz von alpha-Amylase aus Gerste und einem oder mehreren Lysozym Genen bestehen oder diese chimären Genfusionen enthalten, in den Genom von Pflanzenzellen (einschließlich Protoplasten) einsetzt und gegebenenfalls
(b) aus den transformierten Pflanzenzellen (einschließlich Protoplasten) vollständige transformierte Pflanzen regeneriert und gegebenenfalls
(c) von den so erhaltenen transformierten Pflanzen oder deren Nachkommen die gewünschten Pflanzenteile (einschließlich Samen) gewinnt.

Transformierte Pflanzenzellen (einschließlich Protoplasten), ausgenommen Pflanzenzellen von Tabak (Nicotiana tabacum), und Pflanzen (einschließlich Pflanzenteile und Samen), ausgenommen Tabakpflanzen (Nicotiana tabacum), mit einer erhöhten Resistenz gegen Pilze und tierische Schädlinge, welche ein oder mehrere der Lysozym Gen-Konstruktionen enthalten sowie solche transformierte Pflanzenzellen und Pflanzen, welche nach den obigen Verfahren erhältlich sind, gehören ebenfalls zur vorliegenden Erfindung.

Teile der vorliegenden Erfindung sind auch die:
(a) Verwendung von Lysozym Gen-Konstruktionen die aus chimären Genfusionen aus dem TR-Promotor, der Signalpeptid Sequenz von alpha-Amylase aus Gerste und einem oder mehreren Lysozym Genen bestehen oder diese chimären Genfusionen enthalten, von Vektoren und von erfindungsgemäßen transformierten Mikroorganismen, die die Lysozym Gen-Konstruktion enthalten, zur Transformation von Pflanzenzellen (einschließlich Protoplasten) und Pflanzen (einschließlich Pflanzenteilen und Samen) mit dem Ergebnis einer höheren Resistenz gegen Pilze und tierische Schädlinge sowie die
(b) Verwendung der erfindungsgemäß transformierten Pflanzenzellen (einschließlich Protoplasten) und Pflanzen (einschließlich Pflanzenteilen und Samen) zur Erzeugung von Vermehrungsmaterial sowie zur Erzeugung neuer Pflanzen und deren Vermehrungsmaterial und ganz allgemein die
(c) Verwendung der Lysozym Gen-Konstruktion bei der Abwehr und Bekämpfung von Pilzen und tierischen Schädlingen durch die Erhöhung entsprechender Resistenzen.

Eine Anzahl verschiedener Methoden steht zur Verfügung, die Lysozym Gen-Konstruktion in das genetische Material von Pflanzen bzw. Pflanzenzellen einzusetzen. Der Gentransfer kann nach den allgemein üblichen bekannten Methoden erfolgen, wobei der Fachmann die jeweils geeignete Methode ohne Schwierigkeiten ermitteln kann.

Das Ti-Plasmid von Agrobacterium tumefaciens steht als besonders günstiger und breit einsetzbarer Vektor zur Übertragung von fremder DNA in Genome dikotyler und monokotyler Pflanzen zur Verfügung. Die Lysozym Gen-Konstruktion wird in die T-DNA von geeigneten Ti-Plasmiden eingesetzt (z.B. Zambryski et al. 1983) und durch Infektion der Pflanze, Infektion von Blattscheiben oder durch Kokultur von Protoplasten mit Agrobacterium tumefaciens übertragen.

Eine Alternative ist die Inkubation von Lysozym Gen-Konstruktion und Pflanzenprotoplasten (z.B. Davey et al. 1980; Hain et al., 1985; Krens et al., 1982; Paszkowski et al., 1984) in Gegenwart von Polykationen oder Calziumsalzen und Polyethylenglykol.

Die DNA-Aufnahme kann auch zusätzlich durch ein elektrisches Feld (Elektroporation) begünstigt werden (z.B. Fromm et al. 1986).

Die DNA kann in bekannter Weise auch über Pflanzenpollen eingeführt werden, indem Pollen mit physikalisch beschleunigten Partikeln "beschossen" werden, welche die DNA tragen (vgl. EP-A 0 270 356).

Die Regeneration der Pflanzen erfolgt in bekannter Weise mit Hilfe geeigneter Nährmedien (z.B. Nagy und Maliga 1976).

Es kann beispielsweise das Plasmid pSR 2-4 auf Agrobacterium tumefaciens, das z.B. pGV 3850 bzw. Derivate davon enthält (Zambryski et al. 1983) mit üblichen Methoden (z.B. Van Haute et al. 1983) übertragen werden. Der Erfolg der Transformation kann durch Nopalin- oder NPT(II)-Nachweis überprüft werden. Alternativ dazu kann die Lysozym Gen-Konstruktion in einem binearen Vektor (z.B. Koncz und Schell 1986) kloniert und wie oben beschrieben in einen geeigneten Agrobakterium Stamm (Koncz und Schell 1986) transferiert werden. Der resultierende Agrobakterium Stamm, der die Lysozym Gen-Konstruktion in einer auf Pflanzen transferierbaren Form enthält, wird im weiteren zur Pflanzentransformation verwendet.

In einer bevorzugten Ausführungsform wird das isolierte Plasmid pSR 2-4 in üblicher Weise durch direkten Gentransfer auf Pflanzenprotoplasten übertragen (z.B. Hain et al 1985). Dabei kann das Plasmid in zirkulärer, vorzugsweise jedoch in linearer Form, vorliegen.

Bei der Verwendung des Plasmids pSR 2-4 mit Reportergen werden Kanamycin-resistente Protoplasten dann auf Expression von Lysozym überprüft.

Transformierte (transgene) Pflanzen bzw. Pflanzenzellen werden nach den bekannten Methoden, z.B. durch Blattscheiben Transformation (z.B. Horsch et al. 1985) durch Cokultur regenerierender Pflanzenprotoplasten oder Zellkulturen mit Agrobacterium tumefaciens (z.B. Marton et al. 1979, Hain et al. 1985) oder durch direkte DNA Transfektion erzeugt. Resultierende transformierte Pflanzen werden entweder durch Selektion auf die Expression des Reportergens, z.B. durch die Phosphorylierung von Kanamycin-sulfat in vitro (Reis et al. 1984; Schreier et al. 1985) oder durch die Expression der Nopalinsynthase (nach Aerts et al. 1983) oder von Lysozym durch Northern-Blot-Analyse und Western Blot-Analyse nachgewiesen. Das Lysozym kann auch in bekannter Weise mit Hilfe spezifischer Antikörper in transformierten Pflanzen nachgewiesen werden.

Die Kultivierung der transformierten Pflanzenzellen sowie die Regeneration zu vollständigen Pflanzen erfolgt nach den allgemein üblichen Methoden mit Hilfe der jeweils geeigneten Nährmedien.

Sowohl die transformierten Pflanzenzellen als auch die transformierten Pflanzen, welche Lysozyme enthalten, zeigen eine erheblich höhere Resistenz gegen pflanzenpathogene Pilze und tierische Schädlinge, insbesondere gegen Insekten, Milben und Nematoden.

Im Zusammenhang mit der vorliegenden Erfindung bedeutet der Ausdruck "Pflanzen" sowohl vollständige Pflanzen als auch Pflanzenteile, wie Blätter, Samen, Knollen, Stecklinge u.s.w. "Pflanzenzellen" schließen Protoplasten, Zelllinien, Pflanzenkalli usw. ein. "Vermehrungsmaterial" bedeutet Pflanzen und Pflanzenzellen, welche zur Vermehrung der transformierten Pflanzen und Pflanzenzellen verwendet werden können.

Im vorliegenden Zusammenhang bedeutet der Ausdruck "im wesentlichen gleichwirkende DNA-Sequenzen", daß die Erfindung auch solche Modifikationen umfaßt, bei welchen die Funktion der Lysozym Gene bzw. ihrer Teile nicht derart beeinträchtigt ist, daß Lysozym nicht mehr gebildet wird oder der regulatorische Genteil nicht mehr wirksam wird. Entsprechende Modifikationen können durch den Ersatz, die Hinzufügung und/oder die Entfernung von DNA-Abschnitten, einzelner Kodons und/oder einzelner Nukleinsäuren erfolgen.

Bei den erfindungsgemäß verwendbaren Mikroorganismen bedeutet "Mutanten" solche modifizierten Mikroorganismen, welche noch die für die Ausführung der Erfindung wesentlichen Merkmale aufweisen, insbesondere die jeweiligen Plasmide enthalten.

Die vorliegende Erfindung soll anhand der folgenden beispielhaften Ausführungen näher erläutert wrden:

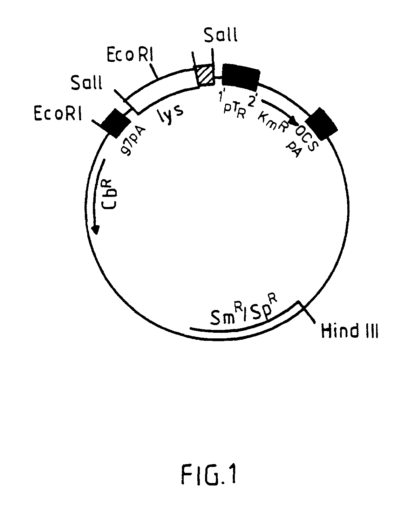

### 1. Beschreibung der verwendeten Plasmidkonstruktion pSR 2-4 (vgl. Figur 1)

Das Plasmid pSR 2-4 enthält eine chimäre Genfusion, die aus dem TR-Promotor (Velten et al. 1984), der Signalpeptid Sequenz von alpha-Amylase aus Gerste und der proteincodierenden Region des T4 Phagenlysozym Gens im Expressionsvektor pAP 2034 (Velten und Schell 1985) konstruiert wurde (K. Düring, Dissertation, Universität Köln 1988). Das Plasmid pSR 2-4 hat die Größe von 9,3 kb.

### Figur 1:

Plasmid pSR2-4 (9,3 kb) kann wie folgt erläutert werden:
- pT_{R}: = Dualer Pflanzenpromotor aus A. tumefaciens
- ocs pA: = Polyadenylierungssequenzregion von Octopinsynthase
- Km^{R}: = Kanamycinresistenzgen aktiv in Planzen
- g7pA: = Polyadenylierungsregion des Gen 7 aus Agrobakterium
- Schraffierter Bereich: = Signalpeptidsequenz von alpha-Amylase aus Gerste
- lys: = kodierende Sequenz für T4 Phagenlysozym
- Cb^{R}: = Carbpenicillin-Resistenzgen
- Sm^{R}: = Streptomycin-Resistenzgen
- Sp^{R}: = Spectomycin-Resistenzgen

Die Lysozym Gen-Konstruktion wurde gemäß der unten erläuterten Methoden beispielsweise auf Tabak und Kartoffel übertragen.

Wie bereits oben dargelegt, wurde der E. coli Stamm TBl pSR 2-4, der das Plasmid pSR 2-4 in leicht isolierbarer Form enthält, bei der Deutschen Sammlung von Mikroorganismen hinterlegt.

### 2. Transformation von Tabak

### a) Kultur von Tabaksprossen und Isolierung von Tabakprotoplasten:

Nicotiana tabacum (Petit Havanna SR1) wird als sterile Sproßkultur auf hormonfreiem LS Medium (Linsmaier und Skoog 1965) vermehrt.

In Abständen von ca. 6-8 Wochen werden Sproßabschnitte auf frisches LS-Medium umgesetzt. Die Sproßkulturen werden bei 12 h Licht (1000-3000 Lux) in einem Kulturraum bei 24-26°C gehalten.

Für die Isolierung von Blattprotoplasten werden ca. 2 g Blätter (ca. 3-5 cm lang) mit einer frischen Rasierklinge in kleine Stücke (0,5 cm x 1 cm) geschnitten. Das Blattmaterial wird in 20 ml Enzymlösung, bestehend aus K3 Medium (Nagy und Maliga 1976), 0,4 M Saccharose, pH 5,6, 2 % Zellulase R10 (Serva), 0,5 % Macerozym R10 (Serva) für 14-16 h bei Raumtemperatur inkubiert. Danach werden die Protoplasten durch Filtration über 0,30 mm und 0,1 mm Stahlsiebe von Zellresten getrennt. Das Filtrat wird 10 Minuten lang bei 100 x g zentrifugiert. Während dieser Zentrifugation flotieren intakte Protoplasten und sammeln sich in einer Bande am oberen Rand der Enzymlösung. Das Pellet aus Zellresten und die Enzymlösung werden mit einer Glaskapillare abgesaugt. Die vorgereinigten Protoplasten werden mit frischem K3 Medium (0,4 M Saccharose als Osmotikum) auf 10 ml aufgefüllt und erneut flotiert. Das Waschmedium wird abgesaugt und die Protoplasten werden für Kultur oder folgende Infektion mit Agrobakterien (Kokultur) auf 1-2 x 10⁵/ml verdünnt. Die Protoplastenkonzentration wird in einer Zählkammer bestimmt.

### b) Transformation von regenerierenden Tabakprotoplasten durch Kokultur mit Agrobacterium tumefaciens:

Es wird im folgenden die Methode von Marton et al. 1979 mit kleinen Veränderungen benutzt. Die Protoplasten werden wie beschrieben isoliert und in einer Dichte von 1-2 x 10⁵/ml in K3 Medium (0,4 M Saccharose, 0,1 mg/l NAA, 0,2 mg Kinetin) 2 Tage im Dunkeln und ein bis zwei Tage lang unter Schwachlicht (500 lux) bei 26°C inkubiert. Sobald die ersten Teilungen der Protoplasten auftreten, werden 30 µl einer Agrobakteriumsuspension in minimal A (Am) Medium (Dichte ca. 10⁹ Agrobakterien/ ml) zu 3 ml regenerierenden Protoplasten gegeben. Die Kokulturdauer beträgt 3-4 Tage bei 20°C im Dunkeln. Danach werden die Tabakzellen in 12 ml Zentrifugenröhrchen gefüllt, mit Seewasser (600 mOsm/kg) auf 10 ml verdünnt und bei 60 x g 10 Minuten lang pelletiert. Dieser Waschvorgang wird noch 1-2 x wiederholt um den größten Teil der Agrobakterien zu entfernen. Die Zellsuspension wird in einer Dichte von 5 x 10⁴/ml in K3 Medium (0,3 m Saccharose) mit 1 mg/l NAA (Naphthyl-1-essigsäure), 0,2 mg/l Kinetin und 500 mg/l des Cephalosporin-Antibiotikums Cefotaxim kultiviert. Die Zellsuspension wird jede Woche mit frischem K3 Medium verdünnt und der osmotische Wert des Mediums graduell um 0,05 m Saccharose (ca. 60 mOsm/kg) pro Woche reduziert. Die Selektion mit Kanamycin (100 mg/l Kanamycinsulfat (Sigma), 660 mg/g aktives Km) wird 2-3 Wochen nach der Kokultur in Agarose Bead Typ Kultur (Shillito et al. 1983) gestartet. Kanamycinresistente Kolonien können 3-4 Wochen nach Beginn der Selektion vom Hintergrund zurückgebliebender Kolonien unterschieden werden.

### c) Direkte Transformation von Tabakprotoplasten mit DNA, Calciumnitrat-PEG Transformation

In einer Petrischale werden ca. 10⁶ Protoplasten in 180 µl K3 Medium mit 20 µl wäßriger DNA Lösung welche 0,5 µg/µl pSR 2-4 enthält, vorsichtig gemischt. Anschließend werden 200 µl Fusionslösung (0,1 M Calciumnitrat, 0,45 M Mannit, 25 % Polyethylenglykol (PEG 6000), pH 9) vorsichtig zugegeben. Nach 15 Minuten werden 5 ml Waschlösung (0,275 M Calciumnitrat pH 6) addiert und nach weiteren 5 Minuten werden die Protoplasten in ein Zentrifugenröhrchen transferiert und bei 60 x g pelliert. Das Pellet wird in einer Meinen Menge K3 Medium aufgenommen und wie im nächsten Abschnitt beschrieben kultiviert. Alternativ können die Protoplasten nach Hain et al. 1985 transformiert werden.

### d) Kultur der mit DNA inkubierten Protoplasten und Selektion Kanamycin resistenter Kalli

Für die im folgenden beschriebene Kultur und Selektion Kanamycin resistenter Kolonien wird eine modifizierte "Bead Type culture"-Technik (Shillito et al. 1983) verwendet. Eine Woche nach Behandlung der Protoplasten mit DNA (vgl. c) werden 3 ml der Zellsuspension mit 3 ml K3 Medium (0,3 M Saccharose + Hormone; 1,2 % (Seaplaque) LMT Agarose (low melting agarose, Marine Colloids) in 5 cm Petrischalen gemischt. Für diesen Zweck wird Agarose trocken autoldaviert und nach Zugabe von K3 Medium im Mikrowellenherd kurz aufgekocht. Nach Erstarren der Agarose werden die Agarosescheiben ("beads") mit den eingebetteten Tabakmikrokalli für weitere Kultur und Selektion in 10 cm Petrischalen transferiert und je 10 ml K3 Medium (0,3 M Saccharose, 1 mg/l NAA, 0,2 mg/l Kinetin) und 100 mg/l Kanamycinsulfat (Sigma) addiert. Das Flüssigmedium wird jede Woche gewechselt. Dabei wird der osmotische Wert des Mediums stufenweise herabgesetzt. Pro Woche wird das Austauschmedium (K3 + Km) um 0,05 M an Saccharose (ca. 60 mOsm) reduziert.

Schema der Selektion kanamycinresistenter Tabakkolonien nach DNA Transformation:

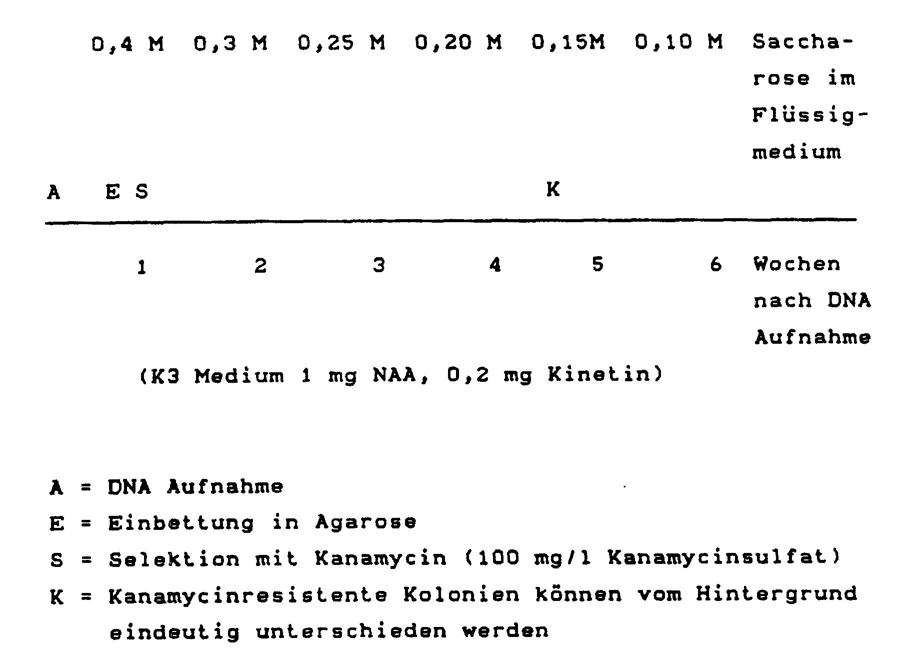

### e) Regeneration kanamycinresistenter Pflanzen

Sobald die kanamycinresistenten Kolonien einen Durchmesser von ca. 0,5 cm erreicht haben, wird die Hälfte auf Regenerationsmedium (LS-Medium, 2 % Saccharose, 0,5 mg/l Benzylaminopurin BAP) gesetzt und bei 12 h Licht (3000-5000 lux) und 24°C im Kulturraum gehalten. Die andere Hälfte wird als Kalluskultur auf LS Medium mit 1 mg/l NAA, 0,2 mg/l Kinetin, 0,1 mg/l BAP und 100 mg/l Kanamycinsulfat propagiert. Wenn die regenerierten Sproße ca. 1 cm groß sind, werden sie abgeschnitten und auf 1/2 LS Medium (1 % Saccharose, 0,8% Agar) ohne Wachstumsregulatoren zur Bewurzelung gesetzt. Die Sproße werden auf 1/2 MS-Medium mit 100 mg/l Kanamycinsulfat bewurzelt und später in Erde umgesetzt.

### f) Transformation von Blattscheiben durch Agrobacterium tumefaciens

Für die Transformation von Blattscheiben (Horsch et al. 1985) werden ca. 2-3 cm lange Blätter von sterilen Sproßkulturen in Scheiben von 1 cm Durchmesser gestanzt und mit einer Suspension entsprechender Agrobacterien (ca. 10⁹/ml) (vgl. b) in Am-Medium, siehen unten) für ca. 5 Minuten inkubiert. Die infizierten Blattstücke werden auf MS-Medium (siehe unten) ohne Hormone für 3-4 Tage bei ca. 24°C gehalten.

Während dieser Zeit überwächst Agrobakterium die Blattstücke. Die Blattstücke werden anschließend in MS-Medium (0,5 mg/ml BAP, 0,1 mg/ml NAA) gewaschen und auf das gleiche Medium (0,8 % Agar) mit 500 µg/ml Cefotaxim (Claforan) und 100 µg/ml Kanamycinsulfat (Sigma) gelegt. Nach zwei Wochen sollte das Medium erneuert werden. Transformierte Sproße werden nach weiteren 2-3 Wochen sichtbar. Die Regeneration von Sproßen sollte parallel auch ohne Selektionsdruck durchgeführt werden. Die regenerierten Sproße müssen dann durch biologische Tests z.B. auf Nopalinsynthase oder Stilbensynthase Aktivität auf Transformation getestet weren. Auf diese Weise werden 1-10% transformierte Sproße erhalten.

### Biochemische Nachweismethode der Transformation

### Nachweis von Nopalin in Pflanzengeweben:

Nopalin wird wie bei Otten und Schilperoort (1978) und Aerts et al. (1979) beschrieben, wie folgt, nachgewiesen. 50 mg Pflanzenmaterial (Kallus oder Blattstücke) werden über Nacht in LS Medium mit 0,1 M Arginin bei Raumtemperatur in einem Eppendorfgefäß inkubiert. Das Pflanzenmaterial wird danach auf saugfähigem Papier abgetupft, in einem frischen Eppendorfzentrifugengefäß mit einem Glasstab homogenisiert und 2 Min. in einer Eppendorfzentrifuge zentrifugiert. 2 µl des Überstandes werden auf ein für Elektrophorese geeignetes Papier (Whatman 3 MM Papier) (20 x 40 cm) punktförmig aufgetragen und getrocknet. Das Papier wird mit dem Laufmittel (5 % Ameisensäure, 15% Essigsäure, 80 % H₂O, pH 1,8) getränkt und bei 400 V für 45 Minuten elektrophoreüsiert. Nopalin läuft zur Kathode hin. Das Papier wird dann mit einem Luftstrom heiß getrocknet und durch Phenanthrenchinon-Färbemittel (gleiches Volumen 0,02 % Phenanthrenchinon in Ethanol und 10 % NaOH in 60 % Ethanol) in Laufrichtung gezogen. Das getrocknete Papier wird unter langweiligem UV-Licht betrachtet und fotografiert. Arginin und Argininderivate werden mit dem Reagenz gelb fluoreszierend gefärbt.

### Neomycin-Phosphotransferase (NPT II) Enzymtest:

NPT II Aktivität in Pflanzengewebe wird durch in situ Phosphorylierung von Kanamycin, wie bei Reiß et al. (1984) beschrieben und von Schreier et al. (1985) modifiziert, wie folgt, nachgeweisen. 50 mg Pflanzengewebe werden in 50 µl Extraktionspuffer (10 % Glycerin, 5 % 2-Mercaptoethanol, 0,1 % SDS, 0,025 % Bromphenolblau, 62,5 mM Tris pH 6,8) unter Zusatz von Glaspulver auf Eis homogenisiert und 10 Minuten lang in einer Eppendorfzentrifuge bei 4°C zentrifugiert. 50 µl des Überstandes werden auf ein natives Polyacrylamidgel (145 x 110 x 1,2 mm; Trenngel: 10 % Acrylamid, 0,33 % Bisacrylamid, 0,375 M Tris pH 8,8, Sammelgel: 5 % Acrylamid, 0.165 % Bisacrylamid, 0,125 M Tris pH 6,8) aufgetragen und über Nacht bei 4°C und 60 V elektrophoretisiert. Sobald der Bromphenolblau-Marker aus dem Gel herausläuft, wird das Gel zweimal mit destilliertem Wasser 10 Min. lang und einmal 30 Min. mit Reaktionspuffer gewaschen (67 mM Tris-Maleat, pH 7,1, 42 mM MgCl₂, 400 mM Ammoniumchlorid). Das Gel wird auf eine gleichgroße Glasplatte gelegt und mit 40 ml 1 %iger Agarose in Reaktionspuffer, der die Substrate Kanamycinsulfat (20 µg/ml) und 20-200 µCi ³²P ATP (Amersham) enthält, überschichtet. Das Sandwichgel wird 30 Min. bei Zimmertempreatur inkubiert und dann ein Blatt Posphozellulosepapier P81 (Whatman) auf die Agarose gelegt. Darüber werden vier Filtrierpapierlagen 3 MM, (Whatman) und einige Papierhandtücher gestapelt. Der Transfer von in situ phorphoryliertem radioaktiven Kanamycinphosphat auf das P81 Papier wird nach 3-4 h gestoppt. Das P81 Papier wird für 30 min. in einer Lösung von Proteinase K und 1 % Natriumdodecyl sulfat (SDS) bei 60°C inkubiert und dann 3-4 mal in 250 ml 10 mM Phosphatpuffer pH 7,5 bei 80°C gewaschen, getrocknet und für 1-12 h lang bei -70°C autoradiografiert (XAR5 Film Kodak).

### Nachweis der T4 Phagenlysozym Genexpression

### a) Isolierung der m-RNA aus Zellkulturen und Pflanzen:

Für die Isolierung von RNA aus Zellkulturen und Pflanzen wurden pro Gramm Pflanzenmaterial 0,1 g steriler Quarzsand, 1 µl β-Mercaptoethanol und 1 ml HO-Puffer (0,4 M Tris/HCl pH 8, 0,1 M NaCl, 0,04 M EDTA, 5 % SDS, bei 65°C) zugegeben und homogenisiert. Es wurden 1 ml Phenol/ Chloroform (1:1) addiert und noch ca. 2 min homogenisiert. Das Homogenat wurde in ein Zentrifugenröhrchen überführt und stark geschüttelt. Nach Zentrifugation (10', 10.000 x g, RT) wurden weitere 2 ml Phenol/Chloroform zugegeben und nach intensivem Schütteln erneut zentrifugiert. Die wäßrige Phase wurde zur Entfernung von Polysacchariden mit 1/4 Volumen Ethanol versetzt, 10 min auf Eis gestellt und dann 10 min bei 10.000 x g und 4°C zentrifugiert. Die Nukleinsäure wurde anschließend mit doppeltem Volumen Ethanol ausgefällt und bei -70°C gelagert. Die gefällte RNA wurde anschließend 2-3 mal mit je 2 ml Natriumacetat (3 M, pH 6) gewaschen. Die RNA wurde in 100 µl sterilem Wasser gelöst.

### b) Elektrophorese von RNA:

Denaturierung der RNA: Für die Elektrophorese von RNA wurden 6,75 µl RNA (20 µg) mit 3 µl 5fach Puffer (0,2 M MOPS, 50 mM Natriumacetat, 5 mM EDTA pH 7), 5,25 µl Formaldehyd (37 %) und 15 µl Formamid (deionisiert) versetzt und 15 min lang bei 56°C denaturiert.

Herstellung von Agarosegelen: 3,5 g Agarose wurden in 200 ml Wasser aufgekocht und nach Abkühlen auf 60°C mit 70 ml 5fach Puffer und 62 ml Formaldehyd versetzt. Die Lösung wurde mit Wasser auf 350 ml Volumen aufgefüllt und in die Gelapparatur eingefüllt. Die denaturierte RNA wurde mit 3 µl Farbmarker und 1 µl Ethidiumbromid (5 mg/ml) gemischt und für 6 h bei 100 V elektrophoretisiert.

### c) Northern Blotting:

Das Gel wurde anschließend 3 mal 10 min lang in sterilem Wasser gewaschen. Die RNA wurde mit einem Standardblottingverfahren (3-4 h in 20 x SSC) auf Nitrocellulosefilter übertragen (Maniatis et al. 1982).

### d) Hybridisierung an Northern blots:

100 ng isoliertes Sal 1-Fragment aus pSR 2-4 wurden mit Nick-Translation radioaktiv markiert (spez. Aktivität > 4 x 10⁸ cpm/µg). Die Hybridisierung erfolgte bei 42°C über Nacht, wie bei Thomzik und Hain 1988 beschrieben.

Mit diesem Verfahren wurde die Synthese von T 4-Phagenlysozym spezifischer mRNA in transformiertem Tabak nachgewiesen.

### Nachweis von T4 Phagenlysozym in Tabak durch Western blotting

Zur Isolierung von Gesamtprotein aus Tabak wurden 100 mg Pflanzenmaterial in doppelt konzentriertem SDS Probenpuffer (150 mM Tris/HCl pH 6,8, 2% SDS, 20% Glycerin, 0,004 % Bromphenolblau, 200 mM DTT, 5 mM Ascorbinsäure, 10 mM CHAPS) homogenisiert und 5 min bei 95°C inkubiert. Nach Zentrifugation (10.000 x g, 5 min) wurde ein Aliquot des Überstandes (10-100 µg Gesamtprotein) auf ein 15 % SDS Polyacrylamidgel aufgetragen und 12 h bei 60 V elektrophoretisiert. Die aufgetrennten Proteine wurden dann durch Elektroblotting (2 h, 0,8-1 A) in Transferpuffer (25 mM Tris base, 192 mM Glycin, 20 % Methanol) auf PVDF Immobilon Membranen transferiert. Die Membran wurde anschließend 30 min in 5 % Rinderserumalbumin (RSA) in PBS inkubiert. Nach dreimaligem Waschen mit 0,1% RSA in PBS wurde die Membran 2 h lang mit einem polyklonalem T4 Lysozym spezifischen Antikörper inkubiert. Nach dreimaligem Waschen (je 5 min) in 0,1 % RSA/PBS wurde die Membran mit einem Gold markierten Antikörper (goat anti rabbit, Auro Probe^{R} Kit, Janssen Chemie) 2 h nach Vorschrift des Herstellers inkubiert. Nach Waschungen in 0,1% RSA in PBS und in Wasser wurde die Membran mit Enhancer/Initiator (1:1) angefärbt.

### 3. Transformation von Solanum tüberosum (Kartoffel)

Die Transformation wurde genau nach dem in der EP-A 0 242 246, Seiten 14 bis 15 angegebenen Weise durchgeführt, wobei die Agrobakterien Ti-Plasmide enthalten, die die Lysozym Gen-Konstruktion des Plasmids pSR 2-4 tragen.

Alle Prozentangaben in den obigen Beispielen beziehen sich auf Gewichtsprozente, wo nichts anderes angegeben wird.

In den gemäß den obigen Beispielen erhaltenen Pflanzenzellen und Pflanzen wurde die Anwesenheit des Lysozym Gens durch Southern Blot Analyse bestätigt. Die Expression des Lysozym Gens wurde durch Northern Blot Analyse, Lysozym mit Hilfe von spezifischen Antikörpern nachgewiesen. Transformierte und nicht-transformierte Pflanzen (zum Vergleich) wurden mit einer Sporensuspension von Botrytis cinera und Alternaria longipes besprüht und nach 1 Woche der Pilzbefall bonitiert. Die transformierten Pflanzen zeigten (gegenüber den nicht transformierten Vergleichspflanzen) eine erhöhte Resistenz gegen Pilzbefall

### Beispiel für den Nachweis der Resistenzerhöhung

Zur Prüfung einer erhöhten Resistenz gegenüber pilzlichen Pflanzenkrankheiten durch das in den Pflanzen synthetisierte Lysozym wurden die Pflanzen mit Pathogenen inokuliert und der Befallsgrad als Parameter herangezogen.

Als Testpathogene dienten Alternaria longipes (Ell. & Everh.) Mason und Botrytis cinerea Pers.

Die Tabakpflanzen werden in Einheitserde (Fa. Balster) in Töpfe (Durchm. 11 cm) getopft und bei 23°C und 70 bis 80 % rel. Luftfeuchte im Gewächshaus bis Versuchsbeginn angezogen. Die Versorgung mit Wasser und Dünger erfolgt nach Bedarf. Zur Inokulation werden die Blätter der 5 bis 8 Wochen alten Pflanzen mit Sporensuspensionen der Pathogene tropfnaß angesprüht. Anschließend werden Pflanzen unter für die Pathogene günstigen Bedingungen bei anfänglich 100 % rel. Luftfeuchte bei 21°C inkubiert. Nach 4 bis 8 Tagen wird der Gesundheitszustand der Pflanzen anhand der befallenen Blattfläche in Prozent ermittelt.

Wie aus den Tabellen (I und II) ersichtlich wird, weisen mit pSR 2-4 gemäß Beispiel 2 transformierte Pflanzen einen geringeren Befall sowohl mit Alternaria longipes als auch mit Botrytis cinerea auf als die des Wildtyps SR₁.

**Tabelle I**

| Einfluß des Gens für Lysozym auf den Befall der Tabakpflanzen mit Alternaria longipes | | | | | | |
|---|---|---|---|---|---|---|
| | % befallene Blattfläche auf Blatt | | | | | |
| Pflanze | 1 | 2 | 3 | 4 | x | Reduktion* |
| SR₁-Wildtyp | 40 | 43 | 35 | 33 | 38 | - |
| Transformierte Pflanze | 14 | 11 | 2 | 2 | 7 | 82% |

| | | | | | | |
|---|---|---|---|---|---|---|
| *) Reduktion berechnet nach Abbott | | | | | | |

**Tabelle II**

| Einfluß des Gens für Lysozym auf den Befall der Tabakpflanzen mit Botrytis cinerea | | | | | | |
|---|---|---|---|---|---|---|
| | % befallene Blattfläche auf Blatt | | | | | |
| Pflanze | 1 | 2 | 3 | 4 | x | Reduktion |
| SR₁-Wildtyp | 100 | 100 | 100 | 100 | 100 | - |
| Transformierte Pflanze | 23 | 35 | 23 | 30 | 28 | 72 % |
| *) Reduktion berechnet nach Abbott | | | | | | |

Im folgenden werden einige der bei der Transformation von Pflanzen bzw. Pflanzenzellen eingesetzte Medien beschrieben:

### Am-Medium

| | |
|---|---|
| 3,5 g | K₂HPO₄ |
| 1,5g | KH₂PO₄ |
| 0,5 g | Na₃ Citrat |
| 0,1g | MgSO₄ x 7H₂O |
| 1 g | (NH₄)₂SO₄ |
| 2 g | Glukose |
| | ad 1 l |

### Medium für sterile Sproßkultur von Tabak

| | | |
|---|---|---|
| Macro-elemente 1/2 der Konzentration der MS Salze | | |
| Micro-elemente 1/2 der Konzentration der MS Salze | | |
| Fe-EDTA | Murashige und Skoog (MS) | |
| Myo-Inosit | | 100 mg/l |
| Sucrose | | 10 mg/l |
| Agar | | 8 g/l |
| Vitamine | Ca-panthotenat | 1 mg/l |
| | Biotin | 10 mg/l |
| | Nicotinsäure | 1 mg/l |
| | Pyridoxin | 1 mg/l |
| | Thiamin | 1 mg/l |
| pH 5,7 vor dem Autoklavieren | | |

### K3-Medium

Zur Kultur von Nicotiana tabacum petit Havana SR1, Nicotiana tabacum Wisconsin 38, und Nictotiana plumbaginifolia Protplasten (Nagy und Maliga, 1976)

| | | |
|---|---|---|
| Macro-elemente | NH₄NO₃ | 250 mg/l |
| | KNO₃ | 2500 mg/l |
| | CaCl₂ . 2H₂O | 900 mg/l |
| | MgSO₄.7H₂O | 250 mg/l |
| | NaH₂PO₄.1 H₂O | 150 mg/l |
| | (NH₄)₂SO₄ | 134 mg/l |
| | CaHPO₄.1H₂O | 50 mg/l |
| Micro-elemente | H₃BNO₃ | 3 mg/l |
| | MnSO₄.1H₂O | 10 mg/l |
| | ZnSO₄.4H₂O | ₂ mg/l |
| | Kl | 0,75 mg/l |
| | Na₂MoO₄.2H₂O | 0,25 mg/l |
| | CUSO₄.5H₂O | 0,025 mg/l |
| | CoCl₂.6H₂O | 0,025 mg/l |
| Fe-EDTA | Na₂EDTA | 37,2 mg/l |
| | FeSO₂.7H₂O | 27,8 mg/l |
| Inosit | | 100 mg/l |
| Sucrose | | 137 g/l (= 0,4 M) |
| Xylose | | 250 mg/l |
| Vitamine | Nicotinsäure | 1 mg/l |
| | Pyridoxin | 1 mg/l |
| | Thiamin | 10 mg/l |
| Hormon | NAA | 1,0 mg/l |
| | Kinetin | 0,2 mg/l |
| pH 5,6 | | |
| Filter sterilisieren | | |

### Linsmaier und Skoog Medium (Linsmaier und Skoog 1965)

Zur Kultur von regenerierenden Protoplasten und für Gewebekultur von Tabaktumoren und Kallus. Linsmaier und Skoog (LS) Medium ist Murashige und Skoog Medium (Murashige und Skoog, 1962) mit den folgenden Modifikationen:
- Thiamin wird in höherer Konzentration eingewogen 0,4 mg/l anstatt 0,1 mg/l;
- Glycin, Pyridoxin und Nicotinsäure fehlen.

| | | |
|---|---|---|
| Macro-elemente | NH₄NOₐ | 1650 mg/l |
| | KNO₃ | 1900 mg/l |
| | CaCl₂.2H₂O | 440 mg/l |
| | MgSO₄.7H₂O | 370 mg/l |
| | KH₂PO₄ | 170 mg/l |
| Micro-elemente | H₃BO₃ | 6,2 mg/l |
| | MnSO₄.1H₂O | 22,3 mg/l |
| | ZnSO₄.4H₂O | 8,6 mg/l |
| | KI | 0,83 mg/l |
| | Na₂MoO₄.2H₂O | 0,25 mg/l |
| | CuSO₄.5H₂O | 0,025 mg/l |
| | CoCl₂.6H₂O | 0,025 mg/l |
| Fe-EDTA | Na₂EDTA | 37,2 mg/l |
| | FeSO₄.7H₂O | 27,8 mg/l |
| Inosit | | 100 mg/l |
| Saccharose | | 30 g/l |
| Agar | | 8 g/l |
| Vitamine | Thiamin | 0,4 mg/l |
| Hormone: | NAA | 1 mg/l |
| | Kinetin | 0,2 mg/l |
| pH 5,7 vor dem Autoldavieren | | |

Zur Transformation von Pflanzen bzw. Pflanzenzellen sowie zu erfindungsgemäß verwendbaren Lysozym Genen kann die folgende Literatur angeführt weren:
Aerts M, Jacobs M, Hernalsteens JP, Van Montagu M, Schell J (1983) Induction and in vitro culture of Arabidopsis thaliana crown gall tumours. Plant Sci Lett. 17: 43-50
Davey MR, Cocking EC, Freeman J, Pearce N, Tudor I (1980) Transformation of Petunia protoplasts by isolated Agrobacterium plasmid. Plant Sci Lett 18: 307-313
K. Düring, Dissertation, Universität Köln, 1988: Wundinduzierbare Expression und Sekretion von T4 Lysozym und monoklonalen Antikörpern in Nicotiana tabacum.
Fromm ME, Taylor LP, Walbot V (1986) Stable transformation of maize after gene transfer by electroporation. Nature 319: 791-793
Hain, R., Stabel, P., Czernilofsky, A.Pp., Steinbiß, H.H., Herrera-Estrella, L., Schell, J. (1985) Uptake, integration, expression and genetic transmission of a selectable chimeric gene by plant protoplasts. Molec Gen Genet 199: 161-168
Horsch RB, Fry JE, Hoffmann NL, Eichholtz D, Rogers SG, Fraley RT (1985) A simple and general method for transferring genes into plants. Science 277: 1229-1231
Krens FH, Molendijk L, Wullems GJ, Schilperoort RA (1982) in vitro transformation of plant protoplasts with Ti-plasmid DNA. Nature 296: 72-74
Koncz C, Schell J (1986) The promotor of T_{L}-DNA gene 5 controls the tissue-specific expression of chimaeric genes carried by a noval type of Agrobacterium linary vector. Mol. Gen. Genet. (1986) 204: 338-396
Linsmaier DM, Skoog F (1965) Organic growth factor requirements of tobacco tissue cultures. Physiol Plant 18: 100-127
Marton L, Wullems GJ, Molendijk L, Schilperoort PR (1979) In vitro transformation of cultured cells from Nicotiana tabacum by Agrobacterium tumefaciens. Nature 277: 1229-131
Nagy Jl, Maliga P (1976) Callus induction and plant regeneration from mesophyll protoplasts of Nicotiana sylvestris. Z Pflanzenphysiol 78: 453-455
Otten LABM, Schilperoort RA (1978) A rapid microscale method for the detection of Lysopin and Nopalin dehydrogenase activities. Biochim biophys acta 527: 497-500
Paszkowski J, Shillito RD, Saul M, Mandak V, Hohn T, Hohn B, Potrykus I (1984) Direct gene transfer to plants. EMBO J 3: 2717-2722
Shillito RD, Paszkowski J. Potrykus l (1983) Agarose plating and Bead type culture technique enable and stimulate development of protoplast-derived colonies in an number of plant species. Pl Gell Rep 2: 244-247
Maniatis T, Fritsch EF, Sambrook J eds. (1982) Molecular cloning, a laboratory manual. Cold Spring Harbor, New York.
J.E. Thomzik and R. Hain (1988), Transfer and segregation of triazine tolerant chloroplasts in Brassica napus L. Theor Appl Genet (1988) 76: 165-171.
Van Haute E, Joos H, Maes M, Worren G, van Montagu M, Schell J. (1983) Inter generic transfer and exchange recombination of restriction fragments cloned in pBR 322: a novel strategy for the reversed genetics of the ti-plasmids of Agrobakterium tumefaciens. EMBO J.: 2 411-417
Velten J, Velten L, Hain R, Schell J (1984) Isolation of a dual plant promotor fragment from the Ti Plasmid of Agrobacterium tumefaciens. EMBO J 12: 2723-2730
Velten J, Schell J (1985) Seiection-expression plasmid vectors for the use in genetic transormation of higher plants. NAR 13: 6981-6998
Zambryski P. Joos H, Genetello G, van Montagu M, Schell J (1983) Ti-plasmid vector for the introduction of DNA into plant cells without altering their normal regeneration capacity, EMBO J 12: 2143-2150.
Bernd Reiss, Roff Sprengel, Hans Will and Heinz Schalter (1984) A new sensitive method for qualitative and quantitative assay of neomycin phosphotransferase in crude cell tracts, GENE 1081: 211-217
Peter H. Schreier, Elisabeth A. Seftor, Jozef Schell and Hans J. Bohnert (1985) The use of nuclear-encoded sequences to direct the light-regulated synthesis and transport of a foreign protein into plant chloroplasts, EMBO J Vol. 4, No 1: 25-32

Weiterhin können die folgenden veröffentlichten Patentanmeldungen aufgeführt werden:
EP-A 116 718
EP-A 159 418
EP-A 120 515
EP-A-120 516
EP-A-172 112
EP-A-140 556
EP-A-174 166
EP-A-122 791
EP-A-126546
EP-A-164 597
EP-A-175 966
WO 84/02913
WO 84/02919
WO 84/02920
WO 83/01176
EP-A-0 270 356

## Patentansprüche

1. Verwendung von Lysozym Gen-Konstruktionen, die Lysozym exprimieren und welche **dadurch gekennzeichnet sind, daß** sie aus chimären Genfusionen aus dem TR-Promotor, der Signalpeptid Sequenz von alpha-Amylase aus Gerste und einem oder mehreren Lysozym Genen bestehen oder diese chimären Genfusionen enthalten, in Pflanzen zur Erhöhung der Resistenz von Pflanzen gegen Pilze und tierische Schädlinge.

2. Verwendung von Lysozym Gen-Konstruktionen, die Lysozym exprimieren und welche **dadurch gekennzeichnet sind, daß** sie aus chimären Genfusionen aus dem TR-Promotor, der Signalpeptid Sequenz von alpha-Amylase aus Gerste und einem oder mehreren Lysozym Genen bestehen oder diese chimären Genfusionen enthalten, zur Erzeugung von Pflanzen mit erhöhter Resistenz gegen Pilze und tierische Schädlinge.

3. Verwendung gemäß den Ansprüchen 1 oder 2 von Lysozym Gen-Konstruktionen mit Lysozym Genen nicht pflanzlichen Ursprungs.

4. Verwendung gemäß den Ansprüchen 1 oder 2 von Lysozym Gen-Konstruktionen mit Hühnereiweiß-Lysozym Genen und/oder T4 Phagen-Lysozym Genen.

5. Verwendung gemäß den Ansprüchen 1 bis 4 der Lysozym Gen-Konstruktion, wie sie auf dem Plasmid pSR 2-4 enthalten ist, hinterlegt in E. coli DSM 5455, bzw. von deren im wesentlichen gleichwirkenden DNA-Sequenzen.

6. Verwendung von Lysozym Gen-Konstruktionen, die Lysozym exprimieren und die **dadurch gekennzeichnet sind, daß** sie aus chimären Genfusionen aus dem TR-Promotor, der Signal peptid Sequenz von alpha-Amylase aus Gerste und einem oder mehreren Lysozym Genen bestehen oder diese Genfusionen enthalten, zur Transformation von Pflanzenzellen (einschließlich Protoplasten) und Pflanzen (einschließlich Pflanzenteilen und Samen) zur Erhöhung der Resistenz gegen Pilze und tierische Schädlinge.

7. Transformierte Pflanzen (einschließlich Pflanzenteilen und Samen), mit einer erhöhten Resistenz gegen Pilze und tierische Schädlinge, welche in ihrem Genom ein oder mehrere Lysozym Gen-Konstruktionen, die Lysozym exprimieren und die **dadurch gekennzeichnet sind, dass** sie aus chimären Genfusionen aus dem TR-Promotor, der Signalpeptid Sequenz von α-Amylase aus Gerste und einem oder mehreren Lysozym-Genen bestehen oder diese Genfusionen enthalten, tragen, wobei die Pflanzen ausgewählt sind aus Getreide, Kartoffel, Hülsenfrüchten, Sojabohnen, Erdnuss, Gemüse, Obst, Tee-, Kakao- und Kaffeesträuchem, Baumwolle sowie Heilpflanzen.

8. Verfahren zur Herstellung transformierter Pflanzen (einschließlich Pflanzenteile und Samen) mit einer erhöhten Resistenz gegen Pilze und tierische Schädlinge gemäß Anspruch 7, **dadurch gekennzeichnet, dass** man
a) ein oder mehrere Lysozym-Gen-Konstruktionen, die Lysozyme exprimieren und die **dadurch gekennzeichnet sind, dass** sie aus chimären Genfusionen aus dem TR-Promotor, der Signalpeptid Sequenz von α-Amylase aus Gerste und einem oder mehreren Lysozym-Genen bestehen oder diese Genfusion enthalten, in das Genom von Pflanzenzellen (einschließlich Protoplasten) einsetzt und
b) aus den transformierten Pflanzenzellen (einschließlich Protoplasten) vollständige transformierte Pflanzen regeneriert und gegebenenfalls
c) von den so erhaltenen transformierten Pflanzen oder deren Nachkommen die gewünschten Pflanzenteile (einschließlich Samen) gewinnt.

9. Verwendung von transformierten Pflanzen (einschließlich Pflanzenteilen und Samen) gemäß Anspruch 7 zur Erzeugung von Vermehrungsmaterial sowie zur Erzeugung neuer Pflanzen und deren Vermehrungsmaterial mit jeweils erhöhter Resistenz gegenüber Pilzen und tierischen Schädlingen.

10. Vermehrungsmaterial von Pflanzen mit erhöhter Resistenz gegenüber Pilzen und tierischen Schädlingen, erhältlich durch die Vermehrung der transformierten Pflanzen gemäß Anspruch 7.

## Claims

1. Use of lysozyme gene constructs which express lysozyme and which are **characterized in that** they consist of chimeric gene fusions of the TR promoter, the signal peptide sequence of alpha-amylase from barley and one or more lysozyme genes or contain these chimeric gene fusions, in plants for increasing the resistance of plants to fungi and animal pests.

2. Use of lysozyme gene constructs which express lysozyme and which are **characterized in that** they consist of chimeric gene fusions of the TR promoter, the signal peptide sequence of alpha-amylase from barley and one or more lysozyme genes or contain these chimeric gene fusions, for producing plants having increased resistance to fungi and animal pests.

3. Use according to Claim 1 or 2 of lysozyme gene constructs with lysozyme genes of non-vegetable origin.

4. Use according to Claim 1 or 2 of lysozyme gene constructs with chicken albumen lysozyme genes and/or T4-phage lysozyme genes.

5. Use according to Claims 1 to 4 of the lysozyme gene construct as it is contained on plasmid pSR 2-4, deposited in E. coli DSM 5455, or its DNA sequences which act essentially in the same sense.

6. Use of lysozyme gene constructs which express lysozyme and which are **characterized in that** they consist of chimeric gene fusions of the TR promoter, the signal peptide sequence of alpha-amylase from barley and one or more lysozyme genes or contain these gene fusions, for transforming plant cells (including protoplasts) and plants (including seeds and parts of plants) for increasing the resistance to fungi and animal pests.

7. Transformed plants (including seeds and parts of plants) which have an increased resistance to fungi and animal pests and which carry in their genome one or more lysozyme gene constructs which express lysozyme and which are **characterized in that** they consist of chimeric gene fusions of the TR promoter, the signal peptide sequence of α-amylase from barley and one or more lysozyme genes or contain these gene fusions, the plants being selected from the group consisting of cereals, potatoes, legumes, soya beans, peanut, vegetables, fruit; tea, cacao and coffee plants; cotton and medicinal plants.

8. Method for producing transformed plants (including seeds and parts of plants) having an increased resistance to fungi and animal pests according to Claim 7, **characterized in that**
(a) one or more lysozyme gene constructs which express lysozymes and which are **characterized in that** they consist of chimeric gene fusions of the TR promoter, the signal peptide sequence of α-amylase from barley and one or more lysozyme genes or contain this gene fusion, are introduced into the genome of plant cells (including protoplasts) and
(b) complete transformed plants are regenerated from the transformed plant cells (including protoplasts), and, if appropriate,
(c) the desired parts of plants (including seeds) are obtained from the resulting transformed plants or progeny thereof.

9. Use of transformed plants (including seeds and parts of plants) according to Claim 7 for producing propagation material, and for producing novel plants and propagation material thereof, in each case having an increased resistance to fungi and animal pests.

10. Propagation material of plants having an increased resistance to fungi and animal pests, obtainable by multiplying transformed plants according to Claim 7.

## Revendications

1. Utilisation de constructions de gènes de lysozyme qui expriment le lysozyme et qui se caractérisent par le fait qu'elles sont constituées par des fusions de gènes chimères constituées par le promoteur TR, par la séquence du peptide signal de l'α-amylase d'orge et par un ou plusieurs gènes de lysozyme ou qu'elles contiennent ces fusions de gènes chimères, dans des plantes pour augmenter la résistance des plantes vis-à-vis de champignons et de parasites animaux.

2. Utilisation de constructions de gènes de lysozyme qui expriment le lysozyme et qui se caractérisent par le fait qu'elles sont constituées par des fusions de gènes chimères constituées par le promoteur TR, par la séquence du peptide signal de l'α-amylase d'orge et par un ou plusieurs gènes de lysozyme ou qu'elles contiennent ces fusions de gènes chimères, pour l'obtention de plantes possédant une résistance supérieure vis-à-vis de champignons et de parasites animaux.

3. Utilisation selon la revendication 1 ou 2 de constructions de gènes de lysozyme avec des gènes de lysozyme qui ne sont pas d'origine végétale.

4. Utilisation selon la revendication 1 ou 2 de constructions de gènes de lysozyme avec des gènes de lysozyme d'ovalbumine et/ou avec des gènes de lysozyme du phage T4.

5. Utilisation selon les revendications 1 à 4 de la construction de gène de lysozyme telle qu'elle est contenue sur le plasmide pSR 2-4 déposé dans E. coli DSM5455, respectivement de ses séquences d'ADN ayant un effet essentiellement identique.

6. Utilisation de constructions de gènes de lysozyme qui expriment le lysozyme et qui se caractérisent par le fait qu'elles sont constituées par des fusions de gènes chimères constituées par le promoteur TR, par la séquence du peptide signal de l'α-amylase d'orge et par un ou plusieurs gènes de lysozyme ou qu'elles contiennent ces fusions de gènes, pour la transformation de cellules de plantes (y compris des protoplastes) et de plantes (y compris des parties de plantes et des semences) pour augmenter la résistance vis-à-vis de champignons et de parasites animaux.

7. Plantes transformées (y compris des parties de plantes et des semences) possédant une résistance supérieure vis-à-vis des champignons et de parasites animaux qui portent dans leur génome une ou plusieurs constructions de gènes de lysozyme qui expriment le lysozyme et qui se caractérisent par le fait qu'elles sont constituées par des fusions de gènes chimères constituées par le promoteur TR, par la séquence du peptide signal de l'α-amylase d'orge et par un ou plusieurs gènes de lysozyme ou qu'elles contiennent ces fusions de gènes, les plantes étant choisies parmi les céréales, les pommes de terre, les légumineuses, les fèves de soja, les noix, les légumes, les fruits, les théiers, les cacaotiers et les caféiers, le coton ainsi que les plantes officinales.

8. Procédé pour la préparation de plantes transformées (y compris des parties de plantes et des semences) manifestant une résistance supérieure vis-à-vis de champignons et de parasites animaux selon la revendication 7, **caractérisé par le fait que**:
(a) On introduit dans le génome de cellules de plantes (y compris des protoplastes) une ou plusieurs constructions de gènes de lysozyme qui expriment des lysozymes et qui se caractérisent **par le fait qu'**elles sont constituées par des fusions de gènes chimères constituées par le promoteur TR, par la séquence du peptide signal de l'α-amylase d'orge et par un ou plusieurs gènes de lysozyme ou qu'elles contiennent ces fusions de gènes chimères, et
(b) à partir des cellules de plantes transformées (y compris des protoplastes), on régénère des plantes transformées complètes, et le cas échéant
(c) à partir des plantes transformées ainsi obtenues ou de leurs descendants, on obtient les parties de plantes désirées (y compris les semences).

9. Utilisation de plantes transformées (y compris des parties de plantes et des semences) selon la revendication 7 pour obtenir un matériel de reproduction, ainsi que pour obtenir de nouvelles plantes et leur matériel de reproduction possédant respectivement une résistance supérieure vis-à-vis de champignons et de parasites animaux.

10. Matériel de reproduction de plantes possédant une résistance supérieure vis-à-vis de champignons et de parasites animaux, que l'on obtient via la reproduction des plantes transformées selon la revendication 7.
